Europäisches Patentamt

European Patent Office

Office européen des brevets

(11) Veröffentlichungsnummer: **0 493 688 A2**

# EUROPÄISCHE PATENTANMELDUNG

(21) Anmeldenummer: **91120626.6**

(22) Anmeldetag: **30.11.91**

(51) Int. Cl.5: **C12S 13/00**, C12N 1/20, C12R 1/01

(30) Priorität: **08.12.90 DE 4039198**

(43) Veröffentlichungstag der Anmeldung:
**08.07.92 Patentblatt 92/28**

(84) Benannte Vertragsstaaten:
**AT BE CH DE DK ES FR GB GR IT LI LU NL SE**

(71) Anmelder: **BOEHRINGER MANNHEIM GMBH**
**Sandhofer Strasse 116**
**W-6800 Mannheim 31(DE)**
Anmelder: **REMMERS CHEMIE GmbH & Co.**
**Postfach 1255**
**W-4573 Löningen(DE)**

(72) Erfinder: **Kaluza, Klaus, Dr.rer.nat.**
**Hochfeldanger 3**
**W-8173 Bad Heilbrunn(DE)**
Erfinder: **Mayr, Ulrich, Dr.rer.nat.**
**Saelweiherstrasse 30**
**W-8122 Penzberg(DE)**
Erfinder: **Zinsmeister, Klaus, Dr.rer.nat.**
**Corvey Strasse 31**
**W-4573 Löningen(DE)**
Erfinder: **Janning, Friedrich**
**Schützenstrasse 5**
**W-4473 Haselünne(DE)**

(54) **Verfahren zur Entfernung von Nitrat aus porösen anorganischen Baustoffen und Schüttgütern und dafür geeignetes Medium.**

(57) Die Erfindung betrifft ein Verfahren und ein Mittel zu Verringerung der Nitratkonzentration in porösen anorganischen Baustoffen durch denitrifizierende Mikroorganismen. Durch dieses Verfahren bzw. Mittel können 60-80 % des Nitratgehaltes im Gestein irreversibel beseitigt werden.

EP 0 493 688 A2

Die Erfindung betrifft ein Verfahren zur Verminderung des Nitratgehalts in porösem Gestein, insbesondere in porösen anorganischen Baustoffen und Schüttgütern durch den Einsatz von denitrifizierenden Mikroorganismen. Außerdem betrifft die Erfindung ein dafür geeignetes Medium. Das Verfahren bzw. Medium findet daher besondere Anwendung auf dem Gebiet der Sanierung/Restaurierung von Bauwerken.

Aus der Praxis der Sanierungstechnik kennt man viele verschiedene Schadensmechanismen an porösen mineralischen Baustoffen wie Ziegel, Natursteine, Putze und Beton. Sämtlichen baustoffzerstörenden Prozessen gemein ist, daß sie an das Vorhandensein von Wasser und Feuchtigkeit im Baustoff gebunden sind. Man unterscheidet dabei den lösenden und den treibenden Angriff. Unter lösendem Angriff versteht man das Eindringen von Feuchtigkeit bzw. Wasser in den Baustoff, wobei in der Feuchtigkeit Schadstoffe wie z. B. $SO_2$, $SO_3$ oder Stickoxide gelöst sind. Diese Schadstoffe bewirken nun im Baustoff eine Auflösung und Zerstörung des Bindemittels, wobei aus den Bindemitteln in einer chemischen Reaktion leicht oder leichter lösliche Salze gebildet werden. Der sogenannte treibende Angriff bezieht sich auf das Vorhandensein von leicht löslichen Salzen in einem mineralischen Baustoff.

Eine häufige Schadensursache im Zusammenhang mit bauschädlichen, meist leicht löslichen Salzen ist auf die Volumenvergrößerung und die damit verbundene Drucksteigerung beim Kristallisationsvorgang zurückzuführen. Dies kann zum einen durch die Kristallisation der Salze oder zum anderen durch die Einlagerung von Wasser in die sich ausbildenen Kristalle geschehen. Dabei können Drücke von mehreren 100 $N/mm^2$ auf die Porenwandungen aufgebaut werden.

Darüber hinaus besitzen solche Salze in der Regel hygroskopische Eigenschaften. Unter der Hygroskopizität versteht man die Eigenschaft von Salzen, Wasser aus der umgehenden Raumluft bei der entsprechenden Luftfeuchtigkeit aufzunehmen. Solche hygroskopischen Salze können z. B. Calciumchlorid, Natriumsulfat, Magesiumsulfat, Ammonium-, Natrium- und Kaliumnitrat sein, wobei die Nitrate besonders hohe hygroskopische Eigenschaften aufweisen. Nitratsalze erhöhen daher die Baustoffeuchte in geradezu extremer Weise. Schon bei Anwesenheit ab 0,3 Gewichtsprozenten dieser Salze muß mit einer erheblichen bis vollständigen Durchtränkung des Baustoffs gerechnet werden.

Außerdem können nitrifizierende, d. h. Salpetersäurebildende Mikroorganismen, die regelmäßig im Gestein anzutreffen sind, den gesteinszerstörenden Prozeß weiter vorantreiben (s. Chemie in unserer Zeit 24, 160-161 (1990); Zeitschrift für Bauinstandhaltung und Denkmalpflege 10, 110-117 (1987)).

Baustoffe, deren Porengefüge einmal vollständig oder zu einem hohen Prozentsatz durchtränkt sind, können mit einfachen Methoden nicht saniert werden. Chloride und Sulfate können durch Fällungsreaktion mit Blei- oder Bariumverbindungen zumindest zum Teil immoblisiert werden.

Nitrate können nicht präzipitiert werden und ergeben unter diesen Bedingungen lediglich lösliche Verbindungen, die ihrerseits wieder hygroskopisch sind und durch Kristallisation Baustoffe zerstören. Um ein stark belastetes Bauwerksteil zu sanieren, ist es in der Regel notwendig, die nitratbelasteten Partien zu entfernen bzw. zurückzuspitzen oder diese Baustoffe vollständig gegen neue auszutauschen. Dies ist insbesondere in der Baudenkmalpflege mit dem Nachteil verbunden, daß historisch wertvolle Substanz verloren geht. Aber auch sanierungsbedürftige Sockel- und Kellerbereiche können nur schwer, wenn überhaupt, im Sinne der Ursachenbehebung saniert werden. Es bieten sich stattdessen zur Zeit nur kaschierende Maßnahmen, wie z. B. Neuverputzen mit sogenannten Sanierputzen, die eine besondere Salzeinlagerungskapazität aufweisen, oder andere Oberflächenbehandlungen an.

Mikrobiologische Maßnahmen zur Nitratbeseitigung wie sie zur Wasseraufbereitung Anwendung finden (z. B. DE 36 01 669, DE 36 05 962, CZ 183 080, CZ 253 810) sind auf Feststoffe nicht übertragbar, da diese Verfahren in Bioreaktoren unter strenger Kontrolle der Außenbedingungen und als kontinuierliche Prozesse verlaufen.

Aufgabe der vorliegenden Erfindung war es daher, ein Verfahren bzw. ein geeignetes Mittel zur Beseitigung der störenden Einflüsse von Nitratsalzen in porösen anorganischen Baustoffen oder Schüttgütern unter gleichzeitigem Erhalt des Mauerwerkes zur Verfügung zu stellen.

Gemäß der vorliegenden Erfindung wird die Aufgabe durch ein Verfahren gelöst, daß dadurch gekennzeichnet ist, daß eine Lösung, welche - bezogen auf das Gesamtvolumen - die folgenden Komponenten enthält:

a) 0,1 - 10 % (w/v) einer oder mehrer organischer Säuren, und/oder einer oder mehrer Mono- und/oder Polyhydroxylverbindungen;

b) 0,1 - 1,0 % (w/v) einer oder mehrer anorganischer Phosphatverbindungen;

c) 0 - 0,1 % (w/v) Molybdän-(Mo-), Eisen-(Fe-) und/oder Kupfer-(Cu-) Salze und ggf.

d) 0,01 - 0,5 % (w/v) organische oder anorganische Stickstoffverbindung

bei einem ph-Wert von ungefähr 5,5 bis 9,0, in einem Temperaturbereich von 5 bis 40° C auf den nitrathaltigen porösen anorganischen Baustoff aufgetragen wird und nach Verringerung der Nitratkonzentration der Baustoff wiederholt in der gleichen Weise behandelt oder direkt versiegelt wird.

Bevorzugt wird der nitrathaltige poröse anorganische Baustoff zusätzlich mit denitrifizierenden Mikroorganismen behandelt. Diese können entweder in Form einer Vorkultur, bevor oder nachdem die oben beschriebene Lösung aufgebracht wurde, mit dem Baustoff in Kontakt gebracht werden. Die Mikroorganismen können aber auch in oben beschriebener Lösung zunächst suspendiert und dann auf den Baustoff gebracht werden.

Unter denitrifizierenden Mikroorganismen sind solche zu verstehen, die in der Lage sind, Nitrat zu Nitrit, $N_2O$ und Luftstickstoff zu reduzieren, wobei die Mikroorganismen, die befähigt sind, vollständig bis zum Stickstoff zu reduzieren, besonders bevorzugt sind. Durch die $NO_3^-$-Reduzierung bis zu gasförmigem $N_2$ ist die Irrevesibilität der Nitratbeseitigung und somit gleichzeitig die Umweltverträglichkeit des Verfahrens gewährleistet.

Beispielsweise können Mikroorganismen der folgenden Gattungen im Sinne der vorliegenden Erfindung verwendet werden: Pseudomonas, Paracoccus, Thiosphaera, Bacillus, Alkaligenes, Thiobacillus, Azospirillum und Rhizobium. Folgende Spezies sind dabei bevorzugt: Thiobacillus denitrificans, Thiosphaera pantotropha, Pseudomonas stutzeri, Pseudomonas fluorescenz, Pseudomonas spec. (P. paucimobilis), Pseudomonas spec. (früher P. nitrificans), Paracoccus denitrificans, Paracoccus halodenitrificans, Bacillus spec., Alkaligenes faecalis, Azospirillum brasilense und Aquaspirillum magnetotacticum. Besonders bevorzugt sind für die erfindungsgemäße Nitratentfernung die Mikroorganismen Thiosphaera pantotropha, ATCC 35512, Pseudomonas spec. (P. paucimobilis), DSM 6291, und Pseudomonas spec., DSM 1650, (früher: P. nitificans).

Die meisten der für die vorliegende Erfindung geeigneten denitrifizierenden Mikroorganismen sind öffentlich zugänglich und beispielsweise bei der DSM, Göttingen, und ATCC, Maryland/USA, hinterlegt sowie - neben anderen - von Payne in Nato Conference Series Vol. 9, 47-65 (1985) beschrieben. Bevorzugt werden die denitrifizierenden Mikroorganismen in einer Zelldichte von $10^3$ bis $10^7$ Keimen/ml als Reinkultur oder als Gemisch in der genannten Lösung suspendiert und als Suspension auf den Baustoff aufgetragen. Die Applikation der Lösung bzw. Suspension kann beispielsweise durch Besprühen, Pinsel oder ähnliche Hilfsmittel oder Injizieren erfolgen.

Die Denitrifizierlösung enthält, ggf. neben dem suspendierten Mikroorganismus, ein definiertes Angebot an Kohlenstoff-, Phosphat- und Stickstoffquellen sowie wesentliche Spurenelemente. Als Kohlenstoffquelle kann eine organische Säure, wie beispielsweise Laktat, Adipat, Succinat, Acetat oder eine höhermolekulare Mono- oder Dicarbonsäure verwendet werden. Außerdem kommen hier mono- bzw. polyfunktionelle Hydroxylverbindungen wie Alkohole oder Zucker, wie z. B. Ethanol, Glycerin, Glucose sowie Extrakte organischer Zusammensetzung wie z. B. Trypton, Pepton oder Hefeextrakt in Betracht. Der Konzentrationsbereich kann zwischen ungefähr 0,1 und 10 Gewichtsprozenten variieren, bevorzugt werden ungefähr 0,5 bis 5 Gewichtsprozente dieser organischen Verbindungen zugegeben.

Als anorganische Phosphatverbindung sind alle gut löslichen Phosphat-Salze, wie z. B. $Na_2HPO_4$ und/oder $KH_2PO_4$, geeignet. Der Phosphatsalz-Gehalt in der Denitrifizierlösung liegt zwischen 0,1 und 1,0 Gewichtsprozenten, vorzugsweise zwischen 0,5 und 0,8 Gewichtsprozenten.

Außerdem ist es erforderlich, dem Medium Spurenelemente zuzusetzen, was beispielsweise in Form von Magnesiumsulfat, Zinksulfat, Calciumchlorid, Manganchlorid, Eisensulfat, Ammoniummolybdat, Kupfersulfat und/oder Kobaltchlorid geschehen kann. Die Gesamtkonzentration liegt hier vorteilhafterweise bei ungefähr 0,1 Gewichtsprozenten, kann jedoch bei der Verwendung von nichtdestilliertem Wasser zur Mediumbereitung und/oder Verwendung von entsprechenden komplettierten Medien für die Vorkultur auch unterbleiben. Außerdem ist es vorteilhaft EDTA zuzugeben.

Desweiteren kann dem Medium eine organische oder anorganische Stickstoffverbindung, wie beispielsweise in Form von Aminosäuren oder Ammoniumsalzen oder Trypton bzw. Hefeextrakt zugesetzt werden. Bevorzugt sollte die Stickstoffverbindung in einem Konzentrationsbereich von 0,01 bis 0,5 Gewichtsprozenten zugesetzt werden.

Mit dem erfindungsgemäßen Verfahren wird nach ungefähr 7 bis 20 Tagen ein um 60 - 80 % verringerter Nitratgehalt im behandelten Baustoff, bis ca. 3 bis 5 cm Tiefe, erreicht. Das Verfahren wird bevorzugt bei annähernd neutralem ph-Wert und Temperaturen von 15 bis 25°C ausgeführt. Es ist jedoch auch möglich, bei niedrigen Temperaturen, bis ca. 5°C, oder bei Temperaturen bis ca. 40°C eine Denitrifizierung durch das erfindungsgemäße Verfahren zu erreichen.

Die Dauer der Behandlung kann, falls erforderlich, zwischen 10 und 60 Tagen variieren. Sie kann z. B. von der Porengröße sowie dem Kontaminationsgrad nitratbildener Bakterien abhängen. Meistens ist die angestrebte Nitratreduzierung bereits nach 7 - 14 Tagen erreicht. In einigen Fällen empfiehlt sich jedoch ein ein- oder mehrmaliges Wiederholen der Behandlung. Anschließend wird der Baustoff - möglichst umgehend, damit die verringerte Hygroskopizität gewährleistet bleibt - nachbehandelt. Dies kann entweder durch zusätzliches Trocknen und Auftragen eines geeigneten Trockenschutzes und/oder durch geeignete Maßnahmen zum Absterben der aufgetragenen Mikroorganismen, wie z. B. das Aufbringen geeigneter Biozide (Antibiotika, die Mikroorganismen hemmende Inhibitoren u. a.), geschehen. In jedem Fall sollte darauf geachtet werden, daß der Baustoff vor dem Versiegeln möglichst trocken ist und nicht-atmungsaktiv (Feuchtigkeit) versiegelt ist. Um der Bausubstanz einen langfristigen Schutz zu geben, kann diese durch zusätzliche Maßnahmen nachbehandelt werden. Solche Maßnahmen sind z. B.: Konsolidierung mit Präparaten auf Kieselsäureesterbasis, wasserabweisende Ausrüstung auf der Basis von Silanen, Siloxanen, Siliconaten oder Beschichtung mittels Mörtel bzw. dispersionsgebundenen Produkten.

Das erfindungsgemäße Verfahren zur Verringerung des Nitratgehaltes ist für alle porösen anorganischen Baustoffe wie Sandstein, Ziegelstein, Tuffstein, Bimsstein, Putze der Mörtelgruppe I - III sowie auf Schüttgüter und Füllstoffe wie Sand, Quarzsand, Bimsmehle und -granulate, Ziegelsplitt und -mehle, Blähton, Vermiculite, Filite und Steinwolle anwendbar.

Die Bestimmung des Nitratgehalts in den Gesteinsproben erfolgt nach geeigneter Extraktion nach bekannten chemischen oder enzymatischen bzw. nach chromatographischen Methoden, wie z. B. HPLC.

Die Anzucht der denitrifizierenden Mikroorganismen erfolgt aerob im Schüttelkolben oder Fermenter bei einer Temperatur von 28 bis 37°C (Vorkultur). Als Nährlösung kann ein handelsübliches Vollmedium wie z. B. Standard 1 - Medium (Merck) verwendet werden.

## Herstellung eines Denitrifikationspräparats

Als Präparat dient eine Nährlösung, welche anorganische Salze wie Phosphate, Chloride, Sulfate der Alkali- und Erdalkalimetalle enthält. Aminosäuren, Peptone, Trypton, Hefeextrakt oder Ammoniak werden als Stickstoffquellen eingesetzt. Zur Unterstützung von Wachstum und Denitrifikation werden Kohlenstoff-Quellen wie Acetat, Succinat,

Adipat, Glycerin, Laktat eingesetzt. Spurenelemente wie Zink, Calcium, Mangan, Eisen, Molybdat, Kupfer, Kobalt werden ebenfalls benötigt.

Zu dieser Lösung werden 1 bis 5 % einer Vorkultur o. g. Mikroorganismen dazugegeben. Die Lebendkeimzahl der fertigen Lösung beträgt $1 \times 10^3$ bis $1 \times 10^7$ pro ml.

Das auf diese Weise hergestellte Denitrifikationspräparat wird auf die zu behandelnden Gesteinsproben aufgetragen. Die benetzten Baustoffe werden bei einer Temperatur von 5 bis 40° C inkubiert.

Die folgenden Beispiele erläutern die Erfindung weiter:
Die Beispiele 1 bis 4 beschreiben den Einsatz von denitrifizierenden Mikroorganismen auf Sandstein-Probekörpern mit geringer Fremdkeimbelastung (< 1 Keim/g Gesteinsmehl).

Die Beispiele 5 bis 7 beschreiben die Nitratentfernung in anorganischen Baustoffen, welche aus sanierungsbedürftigen Objekten stammen. In den Steinen wurde eine natürliche Mikroorganismen-Population ($10$ bis $10^3$ Keime/g Gesteinsmehl) von unterschiedlicher Zusammensetzung beobachtet.

Beispiel 8 beschreibt den Einsatz einer Lösung ohne Verwendung von denitrifizierenden Mikroorganismen zur Nitratreduzierung.

### Beispiel 1

Dieses Beispiel beschreibt den Einsatz von Thiosphaera pantotropha, ATCC 35512 zur Entfernung von Nitrat aus Sandstein.

Probekörpervorbereitung

Quader aus Bentheimer Sandstein (30x30x60 cm) wurden mit 4 l einer 10 %igen Ammoniumnitrat-Lösung getränkt. Die Quader wurden bei einer Temperatur von 15 - 20°C und 30 bis 40 % Luftfeuchtigkeit 21 Tage gelagert.
Von diesen Quadern wurden Bruchstücke von 12x20x10 cm präpariert.

Herstellung des Denitrifizierpräparats

Der Mikroorganismus Thiosphaera pantotropha wurde für 24 h auf 50 ml Standard 1-Medium (handelsübliche Nährlösung von E. Merck, Darmstadt) bei 30°C im 250 ml Schüttelkolben kultiviert. Die Zellen wurden durch Zentrifugation geerntet und in 1 l folgender Lösung resuspendiert: 7.9 g/l $Na_2HPO_4$ x $7H_2O$, 1.5 g/l $KH_2PO_4$, 0.3g/l $NH_4Cl$, 0.1 g/l $MgSO_4$ x $7H_2O$, 0.1 g/L EDTA, 4.4 mg/l $ZnSO_4$, 11 mg/l $CaCl_2$,

EP 0 493 688 A2

10 mg/l $MnCl_2$ x $H_2O$, 10 mg/l $FeSO_4$ x $7H_2O$, 2.2 mg/l $(NH_4)_6$ $Mo_7O_{24}$ x $4H_2O$, 3.1 mg/l $CuSO_4$ x $5H_2O$, 3.2 mg/l $CoCl_2$ x $H_2O$ und 1% Acetat

Applikation auf Baustoff

100 ml der trüben Lösung wurden daraufhin auf eine der Flächen eines Sandsteines von der Größe 12x20x10 cm mit einer Spritzflasche aufgetragen. Die Steinproben wurden anschließend in Polyethylenbeutel bei einer Temperatur von 22 bis 24°C gelagert.

Entnahme der Proben:

Nach bestimmten Zeitabständen wurden Proben entnommen. Dazu wurde eine Scheibe mit einer Dicke von ca. 2 cm abgeschlagen. In einem Mörser wurde dieses Steinbruchstück zu Mehl zerkleinert.

Probenvorbereitung

Das nach Zermahlen der Proben erhaltene Gesteinsmehl (ca. 1 g) wurde mit 1 ml sterilem $H_2O$ (bidest.) versetzt und 30 min unter Schütteln extrahiert. Nach dem Absetzen des mineralischen Materials im Gefäß wurden vom wäßrigen Überstand Aliquots für weitere Analysen entnommen. Das Gewicht der Gesteinsmehlprobe wurde nach Lyophilisation bestimmt.

Analytik

Bestimmung des Anionen-Gehalts:

Der Gehalt an Nitrat, Nitrit und anderen Aninonen wurde durch HPLC-Analyse bzw. im Küvettentest bestimmt. HPLC-Bedingungen:
Verwendete Säule: Polyspher Anion IC AN11 (Merck).
Laufmittel: 4 mM Salicylat-Puffer, ph 7.8.
HPLC-Gerät: LKB
Detektion: indirekte $UV_{254}$-Messung
Küvettentest:
Nitrat-Spectroquant (Merck)
Die Ergebnisse sind in Tabelle 1 zusammengestellt:

Tabelle 1

| Inkubationszeit [d] | 0 | 3 | 7 | 10 | 14 |
|---|---|---|---|---|---|
| Gesamtgehalt [mg Nitrat/g Gesteinsmehl] | 11.6 | 9.8 | 5.8 | 4.6 | 4.7 |
| Restgehalt [% 0-Wert] | 100 | 84 | 50 | 40 | 41 |

Die Ergebnisse belegen eine deutliche Reduktion des Nitratgehaltes in dem Baustoff Sandstein durch den eingesetzten Mikroorganismus.

Beispiel 2

Beispiel 2 beschreibt die Verwendung von Pseudomonas spec. (P. paucimobilis), DSM 6291, zur Entfernung von Nitrat aus Sandstein.
Probekörpervorbereitung, Probenahme, Probenvorbereitung sowie Analytik wurden wie im Beispiel 1 beschrieben ausgeführt.
Bei der Herstellung des Denitrifikationspräparates wurde 1 % Adipat anstelle von Acetat eingesetzt.
Die Ergebnisse sind in Tabelle 2 zusammengestellt:

Tabelle 2

| Inkubationszeit [d] | 0 | 3 | 7 | 14 |
|---|---|---|---|---|
| Gesamtgehalt [mg Nitrat/g Gesteinsmehl] | 11.6 | 4.6 | 4.2 | 2.0 |
| Restgehalt [% 0-Wert] | 100 | 53 | 48 | 23 |

Die Ergebnisse belegen die Eignung von Pseudomonas spec. für die Entfernung von Nitrat in Baustoffen.

Beispiel 3

Beispiel 3 beschreibt die Verwendung von Paracoccus denitrificans, DSM-Nr. 413 zur Entfernung von Nitrat aus Sandstein.

Probekörpervorbereitung etc. wurden wie im Beispiel 1 beschrieben ausgeführt. Bei der Herstellung des Denitrifikationspräparats wurde 1 % Succinat sowie 0.6 % Hefextrakt anstelle von Acetat verwendet.

Die Ergebnisse sind in Tabelle 3 zusammengestellt:

Tabelle 3

| Inkubationszeit [d] | 0 | 10 | 14 | 21 |
|---|---|---|---|---|
| Gesamtgehalt [mg Nitrat/g Gesteinsmehl] | 11.6 | 9.3 | 7.3 | 5.7 |
| Restgehalt [% 0-Wert] | 100 | 80 | 63 | 49 |

Die Ergebnisse belegen die Eignung von Paracoccus denitrificans für die Entfernung von Nitrat in Baustoffen.

Beispiel 4

Beispiel 4 beschreibt den Einsatz eines Gemisches von Thiosphaera pantotropha, ATCC 35512, Pseudomonas stutzeri, ATCC 11607, Pseudomonas spec. (P. paucimobilis), DSM 6291, (jeweils $1\times10^6$ Keime/ml).

Als Probekörper diente eine Platte aus Sandstein (9x18x6 cm), welche mit 425 ml einer 10 %igen Kalium- und Natriumnitratlösung (im Verhältnis 1:1 gemischt) getränkt und anschließend getrocknet wurde.

Die Probennahme, Probenvorbereitung und Analytik wurde wie im Beispiel 1 beschrieben ausgeführt. Jedoch wurden aus den abgeschlagenen Bruchstücken weitere Proben, ausgehend von der benetzten Oberfläche bis in verschiedene Tiefenzonen, präpariert.

Bestimmung der hygroskopischen Feuchtigkeitsaufnahme

Das Gesteinsmehl wurde bei 105ºC bis zur Gewichtskonstanz getrocknet und danach in einem Exsikkator abgekühlt. Auf Petrischalen wurden die Proben anschließend bei 85 % r. F. gelagert. Die Gewichtszunahme wurde im zeitlichen Abstand (1, 4, 7 d etc.) gemessen. Das Ergebnis wird in Gewichtsprozenten, bezogen auf das Trockengewicht, angegeben.

Bestimmung des ph-Wertes

Der ph-Wert des wäßrigen Extraktes wurde mit einem ph-Meter (WTW, Weilheim) bestimmt.

Bestimmung der Lebendkeimzahl

Von den wäßrigen Extrakten wurden Aliquots in steriler 0.85 % NaCl-Lösung verdünnt und die Lebendkeimzahl nach bekannten mikrobiologischen Methoden bestimmt.

Als Nähragar wurde Brain heart infusion agar (Difco Laboratories) verwendet.

Bei der Herstellung des Denitrifikationspräparats wurde 1 % Acetat und 1 % Adipat verwendet.

Die Ergebnisse sind in Tabelle 4 zusammengestellt:

## Tabelle 4

| Inkubations-zeit [d] | 0 | | | 4 | | | 7 | | | 14 | | |
|---|---|---|---|---|---|---|---|---|---|---|---|---|
| Tiefe [cm] | 1,5 | 3 | 6 | 1,5 | 3 | 6 | 1,5 | 3 | 6 | 1,5 | 3 | 6 |
| mg Nitrat/ [g Gesteins-mehl] | 16.3 | 17.3 | 8.9 | 8.3 | 9.8 | 9.5 | 5.4 | 9.1 | 6.2 | 4.4 | 4.5 | 5.3 |
| Gesamtgehalt [mg Nitrat/ Gesteinsmehl] | 14,2 | | | 9,2 | | | 6,9 | | | 4,7 | | |
| Restgehalt [% 0-Wert] | 100 | | | 65 | | | 49 | | | 33 | | |
| Hygroskop. Feuchte [%] | 1.7 | | | 0.8 | | | 0.6 | | | 0.8 | | |
| pH | 7.6 | 7.5 | 7.0 | 8.6 | 8.9 | 7.4 | 8.8 | 7.5 | 7.9 | 8.4 | 8.9 | 9.4 |
| Lebendkeime [$10^x$/g Ge-steinsmehl] | 6 | 5 | 0 | 6 | 7 | 1 | 9 | 8 | 3 | 10 | 9 | 5 |

Die Ergebnisse zeigen auf, daß zur Entfernung von Nitrat auch Gemische von denitrifizierendnen Bakterien geeignet sind. Der Stamm Thiosphaera pantotropha zeigte besonders gutes Wachstum unter diesen Verhältnissen. Als Folge der Nitratentfernung ist eine deutliche Abnahme der hygroskopischen Feuchte zu beobachten.

Beispiel 5

Beispiel 5 beschreibt den Einsatz von denitrifizierenden Bakterien bei einem Ziegelstein belastet mit natürlichen Fremdkeimen.

Versuchsvorbereitung:

Auf einen Ziegelstein (Größe 8x7x12 cm) mit einem Gesamtgehalt an Nitrat von 2.4 mg/g Gesteinsmehl wurden 100 ml eines Bakteriengemisches aufgetragen. Das Gemisch enthielt Pseudomonas spec. (P. paucimobilis), DSM-Nr. 6291, Pseudomonas spec. (früher P. denitrificans), DSM-Nr. 1650 und Thiosphaera pantotropha, ATCC 35512 zu je 1x10$^6$ Keimen pro ml. Das Denitrifikationspräparat enthielt alle Komponenten wie in Beispiel 1 beschrieben, jedoch je 1 % Succinat und Adipat als Kohlenstoffquelle.

Die Ergebnisse sind in Tabelle 5 zusammengefaßt:

Tabelle 5

| Inkubationszeit [d] | 0 | 7 | 21 |
|---|---|---|---|
| Gesamtgehalt [mg Nitrat/g Gesteinsmehl] | 2.4 | 1.5 | 1.1 |
| Restgehalt [% 0-Wert] | 100 | 63 | 46 |

Aus den obigen Werten wird ersichtlich, daß auch in natürlichen Gesteinsproben eine deutliche Entfernung des Nitrates durch den Einsatz von denitrifizierenden Mikroben möglich ist.

Beispiel 6

Beispiel 6 beschreibt den Einsatz von denitrifizierenden Bakterien in natürlichem Kalk-Tuffstein (Größe 20x16x13 cm). Die Versuchsvorbereitung erfolgte wie in Beispiel 5. Der Stein wurde mit 100 ml des Denitrifikationspräparates benetzt.

Die Ergebnisse sind in Tabelle 6 zusammengefaßt.

Tabelle 6

| Inkubationszeit [d] | 0 | 7 | 14 |
|---|---|---|---|
| Gesamtgehalt [mg Nitrat/g Gesteinsmehl] | 3.6 | 1.8 | 1.6 |
| Restgehalt [% 0-Wert] | 100 | 50 | 44 |

Die Ergebnisse belegen die Entfernung von Nitrat aus einem als Baustoff verwendeten Kalk-Tuffstein durch den Einsatz von denitrifizierenden Mikroorganismen.

Beispiel 7

Beispiel 7 beschreibt den Einsatz von denitrifizierenden Bakterien in natürlichem Kalkstein. Auf einen Stein der Größe 18x10x6 cm wurden 50 ml des Bakteriengemisches aufgetragen. Die Versuchsvorbereitung erfolgte wie in Beispiel 5.

Die Ergebnisse sind in Tabelle 7 dargestellt:

Tabelle 7

| Inkubationszeit [d] | 0 | 14 | 21 |
|---|---|---|---|
| Gesamtgehalt [mg Nitrat/g Gesteinsmehl] | 1.4 | 1.1 | 0.5 |
| Restgehalt [% 0-Wert] | 100 | 71 | 36 |

Die Ergebnisse belegen die Entfernung von Nitrat durch denitrifizierende Mikroorganismen aus einem als Baustoff verwendeten Kalkstein.

Beispiel 8

Beispiel 8 beschreibt den Einsatz einer Nährlösung ohne Verwendung von denitrifizierenden Mikroben. Als Probekörper wurde der Ziegelstein, wie in Beispiel 5 beschrieben, mit 100 ml Nährlösung benetzt. Als Nährlösung wurde die in Beispiel 1 eingesetzte Lösung mit je 1 % Succinat und Adipat verwendet.

Die Ergebnisse sind in Tabelle 8 dargestellt:

Tabelle 8

| Inkubationszeit [d] | 0 | 14 | 21 |
|---|---|---|---|
| Gesamtgehalt [mg Nitrat/g Gesteinsmehl] | 2.4 | 1.6 | 1.5 |
| Restgehalt [% 0-Wert] | 100 | 66 | 62 |

Obige Ergebnisse belegen eine Entfernung von Nitrat durch natürlich vorhandene denitrifizierende Keime in den Baustoffen. Durch Zugabe einer geeigneten Nährlösung können einige der vorhandenen Mikroben zur Denitrifikation veranlaßt werden. Jedoch ist die Effizienz der Nitratentfernung weit geringer verglichen mit dem Einsatz von denitrifizierenden Bakterien.

**Patentansprüche**

1. Verfahren zur Verringerung der Nitratkonzentration in porösen anorganischen Baustoffen bzw. Schüttgütern, dadurch gekennzeichnet, daß der poröse Baustoff mit einer Lösung, welche, bezogen auf das Gesamtvolumen, enthält:
   a) 0,1-10 % (w/v) organische Säure und/oder Mono- und/oder Polyhydroxylverbindung;
   b) 0,1-1,0 % (w/v) anorganische Phosphatverbindung;
   c) 0-0,1 % (w/v) Mo-, Fe- und/oder Cu-Salze und ggf.
   d) 0,01-0,5 % (w/v) organische oder anorganische Stickstoffverbindung
   mit einem ph-Wert von ungefähr 3,5 bis 9,0 und in einem Temperaturbereich von 5 bis 40°C behandelt wird und nach Verringerung der Nitratkonzentration die entsprechenden Gesteinspartien versiegelt werden.

2. Verfahren nach Anspruch 1, dadurch gekennzeichnet, daß der Baustoff zusätzlich mit einer Suspension von denitrifizierenden Mikroorganismen in einer Lebendkeimzahl von $10^3$ bis $10^7$/ml behandelt wird.

3. Verfahren nach Anspruch 1 oder 2, dadurch gekennzeichnet, daß als denitrifizierende Mikroorganismen eine oder mehrere der folgenden Bakteriengattungen verwendet werden: Pseudomonas, Paracoccus, Thiosphaera, Bacillus, Alkaligenes, Thiobacillus, Azospirillum und Rhizobium.

4. Verfahren nach den Ansprüchen 1-3, dadurch gekennzeichnet, daß als Mikroorganismen Thiobacillus denitrificans, Thiosphaera pantotropha, Pseudomonas stutzeri, Pseudomonas fluorescenz, Pseudomonas spec., Paracoccus denitrificans, Paracoccus halodenitrificans, Bacillus spec., Alkaligenes faecalis, Azospirillum brasilense und/oder Aquaspirillum magnetotacticum verwendet werden.

5. Verfahren nach den Ansprüchen 2-4, dadurch gekennzeichnet, daß die Mikroorganismen vorkultiviert werden.

6. Verfahren nach den Ansprüchen 2-5, dadurch gekennzeichnet, daß
   a) zunächst die Vorkultur des Mikroorganismus und anschließend die Lösung auf den Baustoff aufgebracht wird,
   b) zunächst die Lösung und anschließend die Vorkultur des Mikroorganismus auf den Baustoff aufgebracht wird oder
   c) der vorkultivierte Mikroorganismus in der Lösung suspendiert wird und anschließend die Suspen-

9

sion auf den Baustoff aufgebracht wird.

7. Verfahren nach den Ansprüchen 1-6, dadurch gekennzeichnet, daß die Baustoffe 10 bis 60 Tage nach der ersten Behandlung mindestens noch einmal nachbehandelt werden.

8. Nährlösung für die Vermehrung und/oder Erhaltung von denitrifizierenden Mikroorganismen, dadurch gekennzeichnet, daß, bezogen auf das Gesamtvolumen, folgende Komponenten enthalten sind:
   a) 0,1-10 % (w/v) organische Säure und/oder mono- und/oder Polyhydroxylverbindung;
   b) 0,1-1,0 % (w/v) anorganische Phosphatverbindung;
   c) 0-0,1 % (w/v) Mo-, Fe- und/oder Cu-Salze und ggf.
   d) 0,01-0,5 % (w/v) organische oder anorganische Stickstoffverbindung
und einen ph-Wert von ungefähr 5,5 bis 7,0 aufweist.

9. Nährlösung nach Anspruch 8, dadurch gekennzeichnet, daß in dieser denitrifizierende Mikroorganismen in einer Lebendkeimzahl von $10^3$ bis $10^7$/ml suspendiert sind.

10. Nährlösung nach Anspruch 8 oder 9, dadurch gekennzeichnet, daß eine oder mehrere Bakteriengattungen der folgenden Gruppe enthalten sind: Pseudomonas, Paracoccus, Thiosphaera, Bacillus, Alkaligenes, Thiobacillus, Azospirillum und Rhizobium.

11. Nährlösung nach den Ansprüchen 8-10 dadurch gekennzeichnet, daß die Mikroorganismen, Thiobacillus denitrificans, Thioshaera pantotropha, Pseudomonas stutzeri, Pseudomonas fluorescenz, Pseudomonas spec., Paracoccus denitrificans, Paracoccus halodenitrificans, Bacillus spec., Alkaligenes faecalis, Azospirillum brasilense und/oder Aquaspirillum magnetotacticum enthalten sind.